# EUROPEAN PATENT SPECIFICATION

(11) **EP 1 962 843 B1**
(45) Date of publication and mention of the grant of the patent: **23.02.2011**
(21) Application number: 06840026.6
(22) Date of filing: 27.11.2006
(51) Int. Cl.: A61K 31/44, A61K 31/4995, A61K 31/517, A61K 45/06

(54) **USE OF PARP-1 INHIBITORS**
VERWENDUNG VON PARP-1-HEMMERN
UTILISATION D'INHIBITEURS DU PARP-1

(30) Priority: 25.11.2005 US 739536 P
(43) Date of publication of application: 03.09.2008
(73) Proprietor: Pharma Mar S.A., Sociedad Unipersonal, 28770 Madrid (ES)
(72) Inventor: SCOTTO, Kathleen, A., Washington Crossing, PA 18977 (US); MANDOLA, Michael, Monmouth Junction, NJ 08852 (US)
(74) Representative: ABG Patentes, S.L.
(86) International application number: PCT/US2006/061254
(87) International publication number: WO 2007/062413

(56) References cited:
- US-A1- 2004 108 086
- US-A1- 2005 020 595
- US-A1- 2005 187 172
- POMMIER Y.: "Topoisomerase-I inhibitors: molecular and cellular determinants of activity"[Online] October 2003 (2003-10), XP002531379 Retrieved from the Internet: URL:http://discover.nci.nih.gov/pommier/To p1.review.pommier.pdf> [retrieved on 2009-06-09]
- BOULTON SALLYANNE ET AL: "Interactive effects of inhibitors of poly(ADP-ribose) polymerase and DNA-dependent protein kinase on cellular responses to DNA damage" CARCINOGENESIS (OXFORD), vol. 20, no. 2, February 1999 (1999-02), pages 199-203, XP002531380 ISSN: 0143-3334
- FAYETTE JÉRÔME ET AL: "ET-743: a novel agent with activity in soft tissue sarcomas." THE ONCOLOGIST 2005 NOV-DEC, vol. 10, no. 10, November 2005 (2005-11), pages 827-832, XP002531381 ISSN: 1083-7159
- DING JIAN ET AL: "Emerging cancer therapeutic opportunities target DNA-repair systems" TRENDS IN PHARMACOLOGICAL SCIENCES, vol. 27, no. 6, June 2006 (2006-06), pages 338-344, XP002531382 ISSN: 0165-6147
- IZBICKA E. ET AL.: 'In vitro antitumor activity of the novel marine agent, Ecteinascidin-743 (ET-743, NSC-648766) against human tumors explanted from patients' ANNALS OF ONCOLOGY vol. 9, no. 9, 1998, pages 981 - 987, XP008127915

## Description

### BACKGROUND OF THE INVENTION

Ecteinascidin-743 (ET-743, Trabectedin, Yondelis®) is a natural marine-based compound derived from the Caribbean tunicate Ecteinascidia turbinata (the sea squirt). Extracts from this organism were shown to have potent cytotoxic activity in the late 1960s, which led to the purification and isolation of individual compounds in the early 1990s. One of these compounds, ET-743, displays potent anti-tumor activity in vitro in a variety of tumor cell lines derived from lung, prostate, ovarian, breast and skin cancers. ET-743 was selected by the NCI for clinical development in 1993 and is currently in Phase III and Phase I combination clinical trials for solid tumors in the US and Europe. Remarkably, ET-743 has shown extraordinary, low dose activity in patients. However, despite considerable data that has accumulated regarding the activities of ET-743, the unique and seemingly novel mechanism(s) of action of this drug are not yet fully elucidated.

Structural modeling studies have shown that ET-743 can undergo covalent interactions with the minor groove of DNA, and that this binding has the unique effect of eliciting bending of the DNA towards the major groove. Studies of the mechanism of action for ET-743 are disclosed in K. Scotto and R. Johnson, "Transcription of the Multidrug Resistance Gene MDR1: A Therapeutic Target," Molecular Interventions, vol. 1, issue 2, pages 117-25 (June 2001); D. Friedman, et al., "Ecteinascidin-743 Inhibits Activated but not Constitutive Transcription," Cancer Research, vol. 62, pages 3377-81 (June 15, 2002); S. Jin et al., "Ecteinascidin 743, a transcription-targeted chemotherapeutic that inhibits MDR1 activation," Proceedings of the National Academy of Sciences of the United States of America, vol. 97, no. 12, pages 6775-79 (June 6, 2000); and K. Scotto, "ET-743: more than an innovative mechanism of action," Anticancer Drugs, 13 Suppl. 1, pages S3-6 (May 2002) .

PARP-1 is an abundant nuclear enzyme which is well characterized as an early sensor of DNA damage which assists in recruiting repair enzymes to lesions sites. It is thought to be one of the first sensors of DNA damage, and upon binding to damaged DNA, the catalytic activity of PARP-1 becomes fully active.

### SUMMARY OF THE INVENTION

The present invention derives from the discovery that the loss of poly (ADP-ribose) polymerase (PARP-1) in a tumor cell population results in increased cellular sensitivity to Ecteinascidin-743 (ET-743).

Therefore, one embodiment relates to a method for improving the cytotoxic effect of Ecteinascidin-743 (ET-743) or an analog thereof on a tumor cell population in a patient said method by administering to the patient, sequentially or simultaneously, a therapeutically effective combination of a composition including ET-743 and an amount of a composition including a PARP-1 inhibitor effective to increase the cytotoxic effect of ET-743 on the tumor cell population.

Another embodiment relates to an anti-tumor composition including a therapeutically effective combination of ET-743 and an amount of a PARP-1 inhibitor effective to increase the cytotoxic effect of ET-743 on the tumor cell population.

An additional embodiment relates to the use of a PARP-1 inhibitor in the manufacture of an anti-tumor medicament characterized by a therapeutically effective amount of ET-743 characterized in that the amount of the PARP-1 inhibitor is effective to increase the tumor cytotoxicity of the ET-743.

Another embodiment further includes combining the ET-743 composition and the PARP-1 inhibitor composition into a single composition prior to administration to the patient.

In another embodiment, the PARP-1 inhibitor is selected from nicotinamide; NU1025; 3-aminobenzamide; 4-amino-1,8-naphthalimide; 1,5-isoquinolinediol; 6(5H)-phenanthriddinone;1,3,4,5,-tetrahydrobenzo(c)(1,6)- and (c)(1,7)-naphthyridin-6-ones; adenosine substituted 2,3-dihydro-1H-isoindol-1-ones; AG14361; AG014699; 2-(4-chlorophenyl)-5-quinoxalinecarboxamide; 5-chloro-2-[3-(4-phenyl-3,6-dihydro-1(2H)-pyridinyl) propyl]-4(3H)-quinazolinone; isoindolinone derivative INO-1001; 4-hydroxyquinazoline; 2-[3-[4-(4-chlorophenyl)-1-piperazinyl]propyl]-4-3(4)-quinazolinone; 1,5-dihydroxyisoquinoline (DHIQ); 3,4-dihydro-5 [4-(1-piperidinyl)(butoxy)-1(2H)-isoquinolone; CEP-6800; GB-15427; PJ34; DPQ; BS-201; AZD2281; BS401; CHP101; CHP102; INH2BP; BSI201; BSI401; TIQ-A; and imidazobenzodiazepines.

In another embodiment, the tumor cell population includes cancer cells selected from lung cancer, prostate cancer, ovarian cancer, breast cancer, skin cancer, and sarcoma.

In another embodiment, the ET-743 composition further includes a pharmaceutically acceptable carrier. In an additional embodiment, the PARP-1 inhibitor composition further includes a pharmaceutically acceptable carrier. In another embodiment, the single composition further includes a pharmaceutically acceptable carrier.

In yet another embodiment, the PARP-1 inhibitor composition is administered two or more times independently selected from before, during, or after the administration of said ET-743 composition.

In one embodiment, the amount of the ET-743 composition is a therapeutically effective amount independent of the amount of said PARP-1 inhibitor composition administered. In another embodiment, the amount of the ET-743 composition is not therapeutically effective when administered without said PARP-1 inhibitor composition.

Another object of the present invention is to provide a method for determining the sensitivity of a tumor or normal cell population in a patient to ET-743 by utilizing polymorphisms and/or mutations in PARP, which result in a loss of PARP activity, in the patient to predict the sensitivity of the cell population to ET-743.

### BRIEF DESCRIPTION OF THE DRAWINGS

FIG. 1a is a plot of percent cellular viability versus ET-743 concentration;
FIG. 1b is a plot of percent cellular viability versus Zalypsis® concentration;
FIG. 2a is a study of cellular viability in the presence of ET-743 and nicotinamide; and
FIG. 2b is a study of cellular viability in the presence of ET-743 and NU1025.

### DETAILED DESCRIPTION OF THE INVENTION

In vitro characterization suggests that ET-743 alkylates minor groove guanine residues. Despite this fairly common attribute of DNA binding drugs, an analysis of ET-743 in over 60 NCI cell lines by COMPARE algorithms showed that ET-743 possesses a unique cytotoxicity profile that demonstrated little correlation with other DNA alkylating agents. Taken together, these data strongly support a novel mechanism of action for ET-743 with respect to DNA.

ET-743 can inhibit the transcriptional activation of a variety of promoters, without inhibiting their constitutive expression. For example, activation of the MDR1 and p21 promoters by a variety of inducers, including the histone deacetylase inhibitor Trichostatin A (TSA), is blocked by treatment with ET-743, while basal levels of transcription from these promoters remains unaffected.

PARP-1 catalyzes the production of ADP-ribose (PAR) polymers, using cellular NAD as a substrate, and adds these highly negatively-charged polymers to acceptor proteins at glutamic acid residues. The addition of PAR polymers to nuclear protein acceptors causes them to dissociate from DNA through electrostatic repulsion. Such dissociation alleviates a strong steric hindrance which allows repair complexes access to the damaged DNA.

While PARP-1 is well known for its role in the DNA damage response; studies of PARP-1 have just recently uncovered its role in gene regulation and other genetic processes under non-pathophysiological conditions. It was recently shown that PARP-1's catalytic activity is potently activated in the presence of nucleosomes as well as bent or cruciform DNA with even greater activation compared to its activation by DNA damage. Moreover, considerable evidence is accumulating which suggests that PARP-1 is involved in the transcriptional activation, and in some cases inhibition, of a variety of promoters in the absence of DNA damage; indeed PARP-1 binding was shown to be an essential step in the formation of RNA polymerase II pre-initiation complexes.

It has now been discovered that the inhibition of PARP-1 in a tumor cell population improves the cytotoxic effect of ET-743 on the cells. Therefore, one embodiment of the present invention includes a method for improving the cytotoxic effect of ET-743 or an analog thereof in a tumor cell population in a patient by administering to the patient a therapeutically effective combination of ET-743 or an analog thereof and an amount of a composition containing a PARP-1 inhibitor effective to increase the cytotoxic effect of the ET-743 or analog thereof on the tumor cell population. The composition containing the PARP-1 inhibitor can be administered before or after the composition containing the ET-743 or analog thereof, or simultaneously therewith. More than one administration of the PARP-1 inhibitor can be performed, so that the PARP-1 inhibitor is administered before and/or during and/or after administration of the composition containing ET-743 or analog thereof.

The amount of ET-743 or analog thereof may be therapeutically effective independent of the amount of PARP-1 inhibitor administered. Alternatively, a subclinical dosage of ET-743 or analog thereof may be administered, for example to reduce side-effects or otherwise improve patient tolerance, and the amount of PARP-1 inhibitor administered is effective to provide a therapeutically effective combination in terms of cytotoxic effect on a tumor cell population.

Suitable PARP-1 inhibitors for use in the present invention include, for example, nicotinamide; NU1025; 3-aminobenzamide; 4-amino-1,8-naphthalimide; 1,5-isoquinolinediol; 6(5H)-phenanthriddinone;1,3,4,5,-tetrahydrobenzo(c)(1,6)- and (c)(1,7)-naphthyridin-6-ones; adenosine substituted 2,3-dihydro-1H-isoindol-1-ones; AG14361; AG014699; 2-(4-chlorophenyl)-5-quinoxalinecarboxamide; 5-chloro-2-[3-(4-phenyl-3,6-dihydro-1(2H)-pyridinyl) propyl]-4(3H)-quinazolinone; isoindolinone derivative INO-1001; 4-hydroxyquinazoline; 2-[3-[4-(4-chlorophenyl)-1-piperazinyl]propyl]-4-3(4)-quinazolinone; 1,5-dihydroxyisoquinoline (DHIQ); 3,4-dihydro-5 [4-(1-piperidinyl)(butoxy)-1(2H)-isoquinolone; CEP-6800; GB-15427; PJ34; DPQ; BS-201; AZD2281; BS401; CHP101; CHP102; INH2BP; BSI201; BSI401; TIQ-A; and imidazobenzodiazepines.

In one embodiment, the tumor cell population includes tumor cells selected from lung cancer, prostate cancer, ovarian cancer, breast cancer, skin cancer, and sarcoma.

Another embodiment includes combining a ET-743 composition and a PARP-1 inhibitor composition into a single composition prior to administration to a patient. In another embodiment, the single composition includes a pharmaceutically acceptable carrier.

In another embodiment, the ET-743 composition includes a pharmaceutically acceptable carrier.

In an additional embodiment, the PARP-1 inhibitor composition includes a pharmaceutically acceptable carrier.

An additional embodiment involves the use of a composition including a PARP-1 inhibitor in the manufacture of a medicament for improving the cytotoxic effect of ET-743 on a tumor cell population.

The term "effective amount" or "therapeutically effective amount" means that amount of a compound or agent that will elicit the biological or medical response of a subject that is being sought by a medical doctor or other clinician. The "effective amount" or "therapeutically effective amount" of ET-743 or an analog thereof includes quantities that would otherwise be insufficient in the absence of a PARP-1 inhibitor.

In practice, the ET-743 composition and/or PARP-1 inhibitor composition may be administered in any variety of suitable forms, for example, topically, parenterally, rectally, or orally. More specific routes of administration include intravenous, intramuscular, subcutaneous, intraocular, intrasynovial, colonical, peritoneal, transepithelial including transdermal, ophthalmic, sublingual, buccal, dermal, ocular, nasal inhalation via insufflation, and aerosol.

The composition(s) may be presented in forms permitting administration by the most suitable route. These compositions may be prepared according to the customary methods, using one or more pharmaceutically acceptable adjuvants or excipients. The adjuvants comprise, inter alia, diluents, sterile aqueous media and the various non-toxic organic solvents. The compositions may be presented in the form of oral dosage forms, or injectable solutions, or suspensions.

The choice of vehicle and ET-743 and/or PARP-1 inhibitors in the vehicle are generally determined in accordance with the solubility and chemical properties of the product, the particular mode of administration and the provisions to be observed in pharmaceutical practice. When aqueous suspensions are used they may contain emulsifying agents or agents which facilitate suspension. Diluents such as sucrose, ethanol, polyols such as polyethylene glycol, propylene glycol and glycerol, and chloroform or mixtures thereof may also be used. In addition, the composition(s) may be incorporated into sustained-release preparations and formulations.

For parenteral administration, emulsions, suspensions or solutions of the composition(s) according to the invention in vegetable oil, for example sesame oil, groundnut oil or olive oil, or aqueous-organic solutions such as water and propylene glycol, injectable organic esters such as ethyl oleate, as well as sterile aqueous solutions of the pharmaceutically acceptable salts, are used. The injectable forms must be fluid to the extent that it can be easily syringed, and proper fluidity can be maintained, for example, by the use of a coating such as lecithin, by the maintenance of the required particle size in the case of dispersion and by the use of surfactants. Prolonged absorption of the injectable compositions can be brought about by use of agents delaying absorption, for example, aluminum monostearate and gelatin. The solutions of the salts of the products according to the invention are especially useful for administration by intramuscular or subcutaneous injection. Solutions of the composition(s) as a free base or pharmacologically acceptable salt can be prepared in water suitably mixed with a surfactant such as hydroxypropyl-cellulose. Dispersion can also be prepared in glycerol, liquid polyethylene glycols, and mixtures thereof and in oils. The aqueous solutions, also comprising solutions of the salts in pure distilled water, may be used for intravenous administration with the proviso that their pH is suitably adjusted, that they are judiciously buffered and rendered isotonic with a sufficient quantity of glucose or sodium chloride and that they are sterilized by heating, irradiation, microfiltration, and/or by various antibacterial and antifungal agents, for example, parabens, chlorobutanol, phenol, sorbic acid, thimerosal, and the like.

Sterile injectable solutions are prepared by incorporating the composition(s) in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze drying technique, which yield a powder of the active ingredient plus any additional desired ingredient from previously sterile-filtered solution thereof.

Topical administration, gels (water or alcohol based), creams or ointments containing the composition(s) may be used. The composition(s) may be also incorporated in a gel or matrix base for application in a patch, which would allow a controlled release of compound through a transdermal barrier.

The percentage of the composition(s) used in the present invention may be varied, it being necessary that it should constitute a proportion such that a suitable dosage shall be obtained. Obviously, several unit dosage forms may be administered at about the same time. A dose employed may be determined by a physician or qualified medical professional, and depends upon the desired therapeutic effect, the route of administration and the duration of the treatment, and the condition of the patient. In the adult, the doses are generally from about 0.001 to about 50, preferably about 0.001 to about 5, mg/kg body weight per day by inhalation, from about 0.01 to about 100, preferably 0.1 to 70, more especially 0.5 to 10, mg/kg body weight per day by oral administration, and from about 0.001 to about 10, preferably 0.01 to 10, mg/kg body weight per day by intravenous administration. In each particular case, the doses are determined in accordance with the factors distinctive to the patient to be treated, such as age, weight, general state of health and other characteristics, which can influence the efficacy of the compound according to the invention.

The composition(s) used in the invention may be administered as frequently as necessary in order to obtain the desired therapeutic effect. Some patients may respond rapidly to a higher or lower dose and may find much weaker maintenance doses adequate. For other patients, it may be necessary to have long-term treatments at the rate of 1 to 4 doses per day, in accordance with the physiological requirements of each particular patient. Generally, the composition(s) may be administered 1 to 4 times per day. Of course, for other patients, it will be necessary to prescribe not more than one or two doses per day.

The following examples set forth hereinbelow illustrate certain aspects of the invention.

### EXAMPLES

### Example 1: Cytotoxicity assays using Ecteinascidin-743 (ET-743, Trabectedin) or Zalypsis® (an analog of Yondelis®)

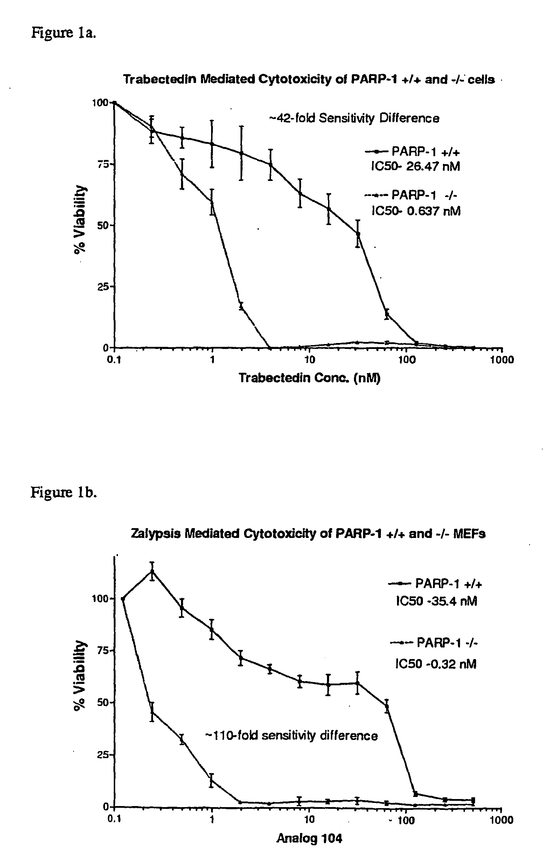

PARP-1 +/+ and -/- mouse embryonic fibroblasts (MEFs) were seeded into 96-well plates at a density of 5,000 cells/well. After 24 hours, cells were treated with serially diluted concentrations of ET-743. 72 hours following the initial treatment, an MTS (3-(4,5-dimethylthiazol-2-yl)-5(3-carboxymethonyphenol)-2-(4-sulfophenyl)-2H-tetrazolium) cytotoxicity assay was performed. Results were plotted as percent cellular viability versus ET-743 or Zalypsis® concentration (FIGS. 1a and 1b, respectively).

As shown in FIG. 1a, loss of PARP-1 results in a -30-fold increase in cellular sensitivity to ET-743. This suggests that changes in PARP-1 activity in tumor cells could influence the efficacy of this drug. Both PARP-1 +/+ and -/- cells died primarily through an apoptotic death pathway as seen by Guava-Nexin analysis (data not shown). A 110-fold increase in sensitivity was seen for PARP-1 -/- cells treated with Zalypsis® (FIG. 1b).

### Example 2: Cytotoxicity assays using nicotinamide or NU1025 in combination with ET-743

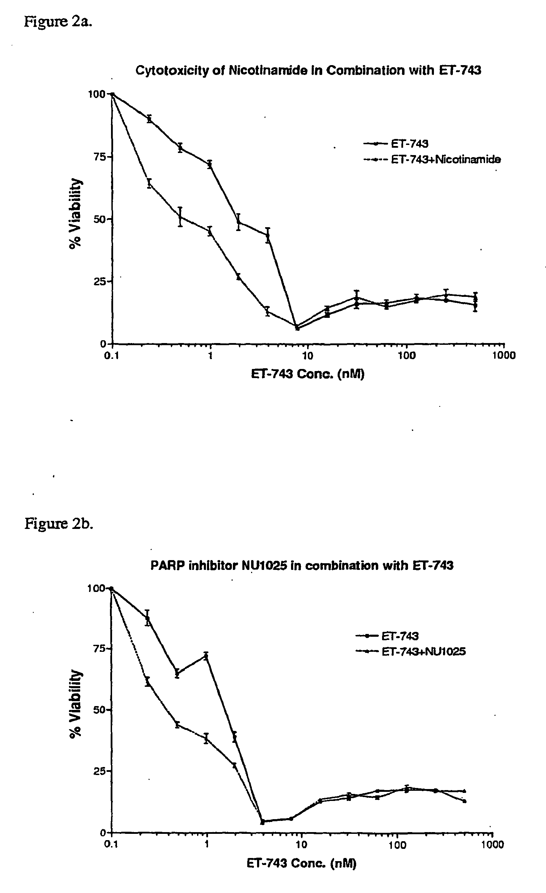

SW620 colon carcinoma cells were pre-treated for 2 hours with either nicotinamide (10 mM) or NU1025 (100 µM). Following the 2 hour pre-treatment, media was washed out and replaced with fresh media containing serially diluted concentrations of ET-743, along with a second fixed dose of PARP inhibitor. Four hours later, media containing ET-743 and the PARP inhibitor was washed out and replaced with another fixed dose of PARP inhibitor. Finally, 4 hours later, a final fixed dose of PARP inhibitor was added. 72 hours following the initial treatment, an MTS cytotoxicity assay was performed. Results were plotted as percent cellular viability versus ET-743 concentration (FIGS. 2a and 2b). IC₅₀ concentrations were: 2 nM with ET-743 alone and 0.41 nM in combination with nicotinamide (FIG. 2a) and 1.8 nM with ET-743 alone and 0.37 nM in combination with NU1025 (FIG. 2b).

As seen in FIG. 2a, treatment with nicotinamide, a general PARP inhibitor, resulted in a 4.9-fold increased sensitization of cells to ET-743. Treatment with a new, more potent and specific PARP inhibitor, NU1025 (up to 1,000 times more potent than nicotinamide) had a similar effect (FIG. 2b) and resulted in a 4.8-fold increase in cellular sensitization to ET-743. Nicotinamide treatment alone resulted in ∼20% cell death. However, treatment with NU1025 alone, the much more potent PARP inhibitor, resulted in no increased level of cytotoxicity above the untreated cells, suggesting that NU1025 is acting synergistically with ET-743 in cell killing.

The foregoing examples and description of the preferred embodiments should be taken as illustrating, the present invention as defined by the claims. As will be readily appreciated, numerous variations and combinations of the features set forth above can be utilized without departing from the present invention as set forth in the claims.

## Claims

1. Use of a PARP-1 inhibitor in the manufacture of a medicament for improving the cytotoxic effect of Ecteinascidin-743 (ET-743) on a tumour cell population, wherein said PAR-1 inhibitor is present in an amount effective to increase the cytotoxic effect of ET-743 on said tumour cell population, and wherein said medicament is administered sequentially or simultaneously with a composition comprising ET-743.

2. Use according to claim 1, wherein the medicament comprising a PARP-1 inhibitor and the composition comprising ET-743 are combined into a single composition.

3. Use according to claims 1 or 2, wherein the PARP-1 inhibitor is selected from the group consisting of nicotinamide; NU1025; 3-aminobenzamide; 4-amino-1,8-naphthalimide; 1,5-isoquinolinediol; 6(5H)-phenanthriddinone; 1,3,4,5,-tetrahydrobenzo(c)(1,6)- and (c)(1,7)-naphthyridin-6-ones; adenosine substituted 2,3-dihydro-1H-isoindol-1-ones; AG14361; AG014699; 2-(4-chlorophenyl)-5-quinoxalinecarboxamide; 5-chloro-2-[3-(4-phenyl-3,6-dihydro-1(2H)-pyridinyl) propyl]-4(3H)-quinazolinone; isoindolinone derivative INO-1001; 4-hydroxyquinazoline; 2-[3-[4-(4-chlorophenyl)-1-piperazinyl]propyl]-4-3(4)-quinazolinone; 1,5-dihydroxyisoquinoline (DHIQ); 3,4-dihydro-5 [4-(1-piperidinyl)(butoxy)-1(2H)-isoquinolone; CEP-6800; GB-15427; PJ34; DPQ; BS-201;BS401; CHP101; CHP102; INH2BP; BSI201; BSI401; TIQ-A; and imidazobenzodiazepines.

4. Use according to any of claims 1 to 3, wherein said tumour cell population comprises cancer cells selected from the group consisting of lung cancer, prostate cancer, ovarian cancer, breast cancer, skin cancer, and sarcoma.

5. Use according to claim 1, wherein the composition comprising ET-743 further comprises a pharmaceutically acceptable carrier.

6. Use according to claim 1, wherein the medicament further comprises a pharmaceutically acceptable carrier.

7. Use according to claim 2, wherein the single composition further comprises a pharmaceutically acceptable carrier.

8. Use according to claim 1, wherein the medicament is administered two or more times independently selected from before, during or after administration of the composition comprising ET-743.

9. Use according to claim 1, wherein the amount of the composition comprising ET-743 is a therapeutically effective amount independent of the amount of the PARP-1 inhibitor administered.

10. Use according to claim 1, wherein the amount of the composition comprising ET-743 is not therapeutically effective when administered without the PARP-1 inhibitor.

11. An anti-tumour composition comprising a therapeutically effective combination of ET-743 and an amount of a PARP-1 inhibitor effective to increase the cytotoxic effect of ET-743 on a tumour cell population.

12. The composition according to claim 11, wherein the amount of the ET-743 is a therapeutically effective amount independent of the amount of the PARP-1 inhibitor administered.

13. The composition according to claim 11, wherein the amount of the ET-743 is not therapeutically effective when administered without the PARP-1 inhibitor.

14. Use of a PARP-1 inhibitor in the manufacture of an anti-tumor medicament **characterized by** a therapeutically effective amount of ET-743 **characterized in that** the amount of said PARP-1 inhibitor is effective to increase the tumor cytotoxicity of said ET-743.

15. The use according to claim 14, wherein said amount of said ET-743 is a therapeutically effective amount independent of the amount of said PARP-1 inhibitor composition administered.

16. The use according to claim 14, wherein said amount of said ET-743 is not therapeutically effective when administered without said PAR-1 inhibitor composition.

## Patentansprüche

1. Verwendung eines PARP-1-Inhibitors bei der Herstellung eines Medikaments zur Verbesserung der cytotoxischen Wirkung von Ecteinascidin-743 (ET-743) auf eine Tumorzellpopulation, wobei der PARP-1-Inhibitor in einer Menge vorhanden ist, die eine Erhöhung der cytotoxischen Wirkung von ET-743 auf die Tumorzellpopulation bewirkt, und wobei das Medikament nacheinander oder gleichzeitig mit einer Zusammensetzung, die ET-743 umfasst, verabreicht wird.

2. Verwendung gemäß Anspruch 1, wobei das Medikament, das einen PARP-1-Inhibitor umfasst, und die Zusammensetzung, die ET-743 umfasst, in einer einzigen Zusammensetzung kombiniert werden.

3. Verwendung gemäß den Ansprüchen 1 oder 2, wobei der PARP-1-Inhibitor aus der Gruppe ausgewählt ist, die aus Nicotinamid, NU1025, 3-Aminobenzamid, 4-Amino-1,8-naphthalimid, 1,5-Isochinolindiol, 6(5H)-Phenanthridinon, 1,3,4,5,-Tetrahydrobenzo(c)(1,6)- und -(c)(1,7)-naphthyridin-6-on, Adenosin-substituierten 2,3-Dihydro-1H-isoindol-1-onen, AG14361, AG014699, 2-(4-Chlorphenyl)-5-chinoxalincarboxamid, 5-Chlor-2-[3-(4-phenyl-3,6-dihydro-1 (2H)-pyridinyl)propyl]-4(3H)-chinazolinon, dem Isoindolinon-Derivat INO-1001, 4-Hydroxychinazolin, 2-[3-[4-(4-Chlorphenyl)-1-piperazinyl]propyl]-4-3(4)-chinazolinon, 1,5-Dihydroxyisochinolin (DHIQ), 3,4-Dihydro-5-[4-(1-piperidinyl)(butoxy)-1(2H)-isochinolon, CEP-6800, GB-15427, PJ34, DPQ, BS-201, BS401, CHP101, CHP102, INH2BP, BSI201, BSI401, TIQ-A und Imidazobenzodiazepinen besteht.

4. Verwendung gemäß einem der Ansprüche 1 bis 3, wobei die Tumorzellpopulation Krebszellen umfasst, die aus der Gruppe ausgewählt sind, die aus Lungenkrebs, Prostatakrebs, Eierstockkrebs, Brustkrebs, Hautkrebs und Sarkomen besteht.

5. Verwendung gemäß Anspruch 1, wobei die Zusammensetzung, die ET-743 umfasst, weiterhin einen pharmazeutisch annehmbaren Träger umfasst.

6. Verwendung gemäß Anspruch 1, wobei das Medikament weiterhin einen pharmazeutisch annehmbaren Träger umfasst.

7. Verwendung gemäß Anspruch 2, wobei die einzelne Zusammensetzung weiterhin einen pharmazeutisch annehmbaren Träger umfasst.

8. Verwendung gemäß Anspruch 1, wobei das Medikament zwei- oder mehrmals zu Zeiten verabreicht wird, die unabhängig aus vor, während oder nach der Verabreichung der Zusammensetzung, die ET-743 umfasst, ausgewählt sind.

9. Verwendung gemäß Anspruch 1, wobei die Menge der Zusammensetzung, die ET-743 umfasst, unabhängig von der verabreichten Menge des PARP-1-Inhibitors eine therapeutisch wirksame Menge ist.

10. Verwendung gemäß Anspruch 1, wobei die Menge der Zusammensetzung, die ET-743 umfasst, nicht therapeutisch wirksam ist, wenn sie ohne den PARP-1-Inhibitor verabreicht wird.

11. Antitumorzusammensetzung, die eine therapeutisch wirksame Kombination von ET-743 und einer Menge eines PARP-1-Inhibitors, die eine Erhöhung der cytotoxischen Wirkung von ET-743 auf eine Tumorzellpopulation bewirkt, umfasst.

12. Zusammensetzung gemäß Anspruch 11, wobei die Menge des ET-743 unabhängig von der verabreichten Menge des PARP-1-Inhibitors eine therapeutisch wirksame Menge ist.

13. Zusammensetzung gemäß Anspruch 11, wobei die Menge des ET-743 nicht therapeutisch wirksam ist, wenn es ohne den PARP-1-Inhibitor verabreicht wird.

14. Verwendung eines PARP-1-Inhibitors bei der Herstellung eines Antitumormedikaments, das durch eine therapeutisch wirksame Menge von ET-743 **gekennzeichnet ist, dadurch gekennzeichnet, dass** die Menge des PARP-1-Inhibitors eine Erhöhung der Tumorcytotoxizität des ET-743 bewirkt.

15. Verwendung gemäß Anspruch 14, wobei die Menge des ET-743 unabhängig von der verabreichten Menge der PARP-1-Inhibitor-Zusammensetzung eine therapeutisch wirksame Menge ist.

16. Verwendung gemäß Anspruch 14, wobei die Menge des ET-743 nicht therapeutisch wirksam ist, wenn es ohne die PARP-1-Inhibitor-Zusammensetzung verabreicht wird.

## Revendications

1. Utilisation d'un inhibiteur de la PARP-1 dans la fabrication d'un médicament destiné à améliorer l'effet cytotoxique de l'ectéinascidine 743 (ET-743) sur une population de cellules tumorales, dans laquelle ledit inhibiteur de la PARP-1 est présent en une quantité efficace pour augmenter l'effet cytotoxique de ET-743 sur ladite population de cellules tumorales, et dans laquelle ledit médicament est administré séquentiellement ou simultanément avec une composition comprenant ET-743.

2. Utilisation selon la revendication 1, dans laquelle le médicament comprenant un inhibiteur de la PARP-1 et la composition comprenant ET-743 sont combinées en une composition unique.

3. Utilisation selon les revendications 1 ou 2, dans laquelle l'inhibiteur de la PARP-1 est sélectionné dans le groupe constitué par le nicotinamide ; NU1025; le 3-aminobenzamide ; le 4-amino-1,8-naphtalimide ; le 1,5-isoquinolinediol ; la 6(5H)-phénanthridinone ; les 1, 3, 4, 5,-tétrahydrobenzo(c)(1,6)- et (c)(1,7)-naphtyridin-6-ones ; les 2,3-dihydro-1H-isoindol-1-ones substituées par l'adénosine ; AG14361 ; AG014699 ; le 2-(4-chlorophényl)-5-quinoxalinecarboxamide ; le 5-chloro-2-[3-(4-phényl-3,6-dihydro-1(2H)-pyridinyl)propyl]-4(3H)-quinazolinone ; le dérivé d'isoindolinone INO-1001 ; Ia 4-hydroxyquinazoline ; la 2-[3-[4-(4-chlorophényl)-1-pipérazinyl]propyl]-4-3(4)-quinazolinone ; I a 1 , 5-dihydroxyisoquinoline (DHIQ) ; la 3,4-dihydro-5 [4-(1-pipéridinyl)(butoxy)-1(2H)-isoquinolone; CEP-6800 ; GB-15427 ; PJ34 ; DPQ ; BS-201; BS401; CHP101 ; CHP102 ; INH2BP ; BSI201 ; BSI401 ; TIQ-A et les imidazobenzodiazépines.

4. Utilisation selon l'une quelconque des revendications 1 à 3, dans laquelle ladite population de cellules tumorales comprend des cellules cancéreuses sélectionnées dans le groupe constitué par le cancer du poumon, le cancer de la prostate, le cancer des ovaires, le cancer du sein, le cancer de la peau et le sarcome.

5. Utilisation selon la revendication 1, dans laquelle la composition comprenant ET-743 comprend en outre un vecteur pharmaceutiquement acceptable.

6. Utilisation selon la revendication 1, dans laquelle le médicament comprend en outre un vecteur pharmaceutiquement acceptable.

7. Utilisation selon la revendication 2, dans laquelle la composition unique comprend en outre un vecteur pharmaceutiquement acceptable.

8. Utilisation selon la revendication 1, dans laquelle le médicament est administré à deux reprises ou plus sélectionnées indépendamment parmi les choix suivants : avant, pendant ou après l'administration de la composition comprenant ET-743.

9. Utilisation selon la revendication 1, dans laquelle la quantité de la composition comprenant ET-743 est une quantité thérapeutiquement efficace indépendante de la quantité de l'inhibiteur de PARP-1 administré.

10. Utilisation selon la revendication 1, dans laquelle la quantité de la composition comprenant ET-743 n'est pas thérapeutiquement efficace lorsqu'elle est administrée sans l'inhibiteur de la PARP-1.

11. Composition anti-tumorale comprenant une combinaison thérapeutiquement efficace de ET-743 et une quantité d'un inhibiteur de la PARP-1 efficace pour augmenter l'effet cytotoxique de ET-743 sur une population de cellules tumorales.

12. Composition selon la revendication 11, dans laquelle la quantité de ET-743 est une quantité thérapeutiquement efficace indépendante de la quantité de l'inhibiteur de la PARP-1 administré.

13. Composition selon la revendication 11, dans laquelle la quantité de ET-743 n'est pas thérapeutiquement efficace lorsqu'elle est administrée sans l'inhibiteur de la PARP-1.

14. Utilisation d'un inhibiteur de la PARP-1 dans la fabrication d'un médicament anti-tumoral **caractérisé par** une quantité thérapeutiquement efficace de ET-743 **caractérisée en ce que** la quantité dudit inhibiteur de la PARP-1 est efficace pour augmenter la cytotoxicité tumorale de ladite ET-743.

15. Utilisation selon la revendication 14, dans laquelle ladite quantité de ladite ET-743 est une quantité thérapeutiquement efficace indépendante de la quantité de ladite composition d'inhibiteur de la PARP-1 administrée.

16. Utilisation selon la revendication 14, dans laquelle ladite quantité de ladite ET-743 n'est pas une quantité thérapeutiquement efficace lorsqu'elle est administrée sans ladite composition d'inhibiteur de la PARP-1.
